# EUROPEAN PATENT APPLICATION

(11) **EP 2 716 279 A1**
(43) Date of publication of application: **09.04.2014**
(21) Application number: 12187269.1
(22) Date of filing: 04.10.2012
(51) Int. Cl.: A61K 9/08, A61K 47/10, A61K 31/47, A61K 9/00

(54) **Oral solutions containing leukotriene antagonists**

(71) Applicant: Lamda UK Ltd., Egham, Surrey TW20 9EY (GB)
(72) Inventor: Karatzas, Angelos, 15235 Vrilissia, Attika (GR)
(74) Representative: Roukounas, Dimitrios

(57) **Abstract**

Stable oral pharmaceutical solutions comprising montelukast sodium as active ingredient, a combination of two alcohols, namely propylene glycol and ethanol, in certain concentration ranges and a pharmaceutically acceptable buffer, wherein the pH of the solution is between 7.5 and 9.0.

## Description

### BACKGROUND OF THE INVENTION

The present invention refers to stable oral pharmaceutical solutions that contain the active substance montelukast sodium.

### BACKGROUND OF THE INVENTION

Montelukast (Formula I) was first disclosed in US 5565473. This document discloses compounds that act as antagonists of leukotriene receptors among which include montelukast.

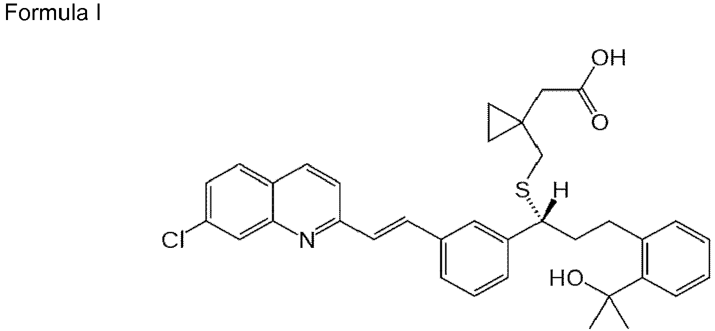

The sodium salt of montelukast, named montelukast sodium, is the active substance of Singulair® medicinal products and it is indicated in the treatment of asthma as add-on therapy (along with inhaled steroids) and in the prophylaxis of asthma in which the predominant component is exercise-induced bronchoconstriction.

Montelukast sodium is soluble in ethanol, methanol and water and practically insoluble in acetonitrile. It is an unstable substance under acidic and oxidizing conditions. When exposed to air it takes up moisture easily, which makes it sensitive to such an environment. The reaction of montelukast with atmospheric oxygen leads to the formation of sulfoxide impurities. Conversely, there is slower rate of decomposition of montelukast in alkaline environment.

According to the patent application US 2006/0147482 unbuffered solutions containing montelukast sodium are not stable even in basic pH ranges of 7 to 11. According to said document, stable solutions containing montelukast sodium can be prepared by using buffers which are able to maintain the pH of the solution between 7 and 11. These solutions exhibit stability with regard to montelukast sodium concentration over time under accelerated storage conditions. Although exact concentrations of excipients are not mentioned, some aqueous solutions comprising propylene glycol, sodium benzoate, sodium saccharin and phosphate buffer are revealed through the examples of said document. The pH of the solutions in the examples is adjusted to the range of 9 to 10.

According to the patent application US 2007/0184101, solid formulations (tablets, chewable tablets) of montelukast are subject to decomposition during the manufacturing process and storage.

According to the patent application US 2009/124657, during the manufacturing process of solid formulations of montelukast, the active substance is prone to a series of reactions that generate impurities (chemical by-products, degradation products). The main known impurities are mok-3 sulfoxide and styrene.

The sensitivity of montelukast even in solid formulations poses a challenge in the development of stable pharmaceutical compositions, especially oral solutions, containing montelukast sodium. Oral solutions present certain advantages over solid pharmaceutical compositions, including flexible dose titration and patient compliance.

Treatment with montelukast requires dosing differentiation, attributed to the involved age groups and the disease's severity. The pharmaceutical form of oral solution enables dose titration, allowing for the adequate coverage of all the dose requirements by adjusting the treatment regimen requirements to the necessary solution volume.

Furthermore, increased adherence to treatment recommendations is achieved by the oral solution formulations owing to the patient-friendly nature of the drug product. This gains great importance when age-sensitive populations such as elderly and children are involved in these regimens. These patients can take advantage of liquid pharmaceutical forms, such as oral solutions, resulting in more effective amelioration of their clinical condition.

A major challenge is posed when oral solutions prone to atmospheric oxidation are supplied in multidose containers which - by nature of their physical form and chemical composition - due to repeated opening and closing, may pose a risk to their content with regard to microbiological contamination, proliferation and/or physicochemical degradation once the closure system has been breached.

Technically more challenging is the development of paediatric formulations, particularly those suitable for very young children. The most important problem is the selection of minimum non-toxic excipients at the lowest feasible concentrations without compromising the stability of the formulations. In other words, low concentrations of excipients help to reduce the potential for toxicological effect to paediatric population, but low concentrations may be insufficient to achieve the required level of stability.

It is obvious that it is to the patient's benefit the development of oral pharmaceutical solutions containing montelukast sodium, which exhibit excellent shelf life and in-use shelf life.

### SUMMARY OF THE INVENTION

The present invention provides oral pharmaceutical solutions, with excellent stability, comprising montelukast sodium in association with a pharmaceutically acceptable liquid carrier.

The oral pharmaceutical solutions according to the invention comprise montelukast sodium as active substance and a liquid carrier which comprises a combination of two alcohols, namely propylene glycol and ethanol, in certain concentration ranges and a pharmaceutically acceptable buffer, where the pH of the solution is between 7.5 and 9.0.

Preferably, the concentration of montelukast sodium in the compositions of the present invention is from 0.5% to 5%, most preferably is from 1% to 2.5%.

The oral pharmaceutical solutions of montelukast sodium according to the invention may be supplied in single-dose or multidose containers.

The oral pharmaceutical solutions of montelukast sodium according to the invention present excellent stability while at the same time allow the optimal selection of concentration of excipients at the lowest feasible level.

The oral pharmaceutical solutions of montelukast sodium according to the invention may be free of additional ingredients commonly used in the preparation of oral liquid pharmaceutical compositions, such as additional antimicrobial agents, and antioxidants, which may raise additional safety and toxicity issues. Absence of additional antimicrobial agents and antioxidants is important particularly in case of paediatric formulations.

The oral pharmaceutical solutions of montelukast sodium according to the invention may also optionally contain additional ingredients commonly used in the preparation of oral liquid pharmaceutical compositions, such as viscosity adjusting agents, non-sugar based artificial sweetening agents, flavoring agents and the like.

The oral pharmaceutical solutions of montelukast sodium according to the invention exhibit excellent stability and extended lifetime. Specifically, they remain physicochemically stable when stored for at least three months at temperature of 40°C and relative humidity of 75 per cent, with the main impurity (sulfoxide) being less than 0.5% by weight relative to the initial amount of montelukast sodium and with the total impurities being less than 1.0%.

The oral pharmaceutical solutions of montelukast sodium according to the invention also exhibit excellent stability and extended in-use shelf life when supplied in multidose containers. Specifically, they remain physicochemically stable when stored at room temperature of 20°C - 25°C and the containers are uncovered at least once a day for at least three months without a precipitate forming, with the main impurity (sulfoxide) being less than 0.5% by weight relative to the initial amount of montelukast sodium and with the total impurities being less than 1.0%.

### DETAILED DESCRIPTION OF THE INVENTION

According to the present invention there is provided pharmaceutical solutions suitable for oral administration with excellent shelf life and in-use shelf life.

In-use shelf life, as defined through European Medicines Agency document named "Note for Guidance on the in-use stability testing of human medicinal products" (CPMPQWP/2934/99), is a period of time during which a multidose product can be used whilst retaining quality within an accepted specification once the container is opened.

As used throughout the present description and claims, the term "%" when referring to the active substance or inactive substances (excipients), means the grams of active or inactive substance per 100ml of solution.

Solutions comprising montelukast sodium, alcohols, such as propylene glycol, ethanol or glycerol, as well phosphoric buffers were included in a sum of preliminary studies. The solutions were filled into glass bottles under nitrogen atmosphere and sealed tightly with a screw cap. The excipients used are those disclosed in the examples of US 2006/0147482. Details of these compositions are presented in the following tables (Tables 1 to 4).

These compositions were studied in relation to their stability at accelerated conditions of temperature (40°C) and relative humidity (75 per cent) for three months. The quantification of the major impurity (sulfoxide) and the total impurities in the compositions before and after the storage period of three months was carried out by HPLC (Tables 1 & 2).

**Table 1**

| **Active substance** | % w/v | | | | |
|---|---|---|---|---|---|
| Montelukast sodium | 0.1037 | 0.1037 | 0.2074 | 0.2074 | 0.2074 |
| corresp. to montelukast acid | 0.1 | 0.1 | 0.2 | 0.2 | 0.2 |

| **Excipients** | | | | | |
|---|---|---|---|---|---|
| Propylene glycol | 5 | 15 | 30 | 40 | 50 |
| Sodium saccharin | 1 | 1 | 1 | 1 | 1 |
| Sodium benzoate | 0.2 | 0.1 | 0.1 | 0.1 | 0.1 |
| Buffer solution (pH 9.0) | q.s.1 ml | q.s.1 ml | q.s.1 ml | q.s.1 ml | q.s.1 ml |
| **% sulfoxide (initial)** | 0.2 | 0.1 | 0.1 | 0.2 | .0.1 |
| **% sulfoxide (3 months)** | 1.3 | 0.9 | 1.0 | 1.1 | 1.1 |
| **% total impurities (initial)** | 0.4 | 0.4 | 0.5 | 0.4 | 0.5 |
| **% total impurities (3 months)** | 4.7 | 2.3 | 3.4 | 3.9 | 3.7 |

**Table 2**

| **Active substance** | % w/v | | | | |
|---|---|---|---|---|---|
| Montelukast sodium | 0.1037 | 0.1037 | 0.2074 | 0.2074 | 0.2074 |
| corresp. to montelukast acid | 0.1 | 0.1 | 0.2 | 0.2 | 0.2 |

| **Excipients** | | | | | |
|---|---|---|---|---|---|
| Ethanol | 5 | 15 | 15 | - | - |
| Glycerol | - | - | 20 | 20 | 40 |
| Sodium saccharin | 1 | 1 | 1 | 1 | 1 |
| Sodium benzoate | 0.2 | 0.1 | 0.1 | 0.1 | 0.1 |
| Buffer solution (pH 9.0) | q.s.1 ml | q.s.1 ml | q.s.1 ml | q.s.1 ml | q.s.1 ml |
| **% sulfoxide (initial)** | 0.2 | 0.2 | 0.1 | 0.2 | 0.2 |
| **% sulfoxide (3 months)** | 1.5 | 1.1 | 0.9 | 2.2 | 2.1 |
| **% total impurities (initial)** | 0.5 | 0.4 | 0.5 | 0.5 | 0.5 |
| **% total impurities (3 months)** | 6.1 | 4.2 | 3.2 | 4.0 | 4.8 |

All resulting solutions were proved to show high levels of known and unknown impurities after three months of storage at temperature of 40°C and relative humidity of 75 per cent, indicating that montelukast sodium decomposes in the selected carriers.

Samples of the solutions described in Tables 1 and 2 were subjected to three-month studies simulating actual in-use conditions in order to address potential in-use stability related issues, following the requirements, as reflected in European Medicines Agency document CPMPQWP/2934/99.

The test simulated the use of the product in practice since the bottles were uncovered once a day and a certain quantity of solution was removed each time. The quantity of removed solution has been chosen so that enough solution for performing all analytical tests at initial and final time points is left in the bottles (Tables 3 & 4).

**Table 3**

| **Active substance** | % w/v | | | | |
|---|---|---|---|---|---|
| Montelukast sodium | 0.1037 | 0.1037 | 0.2074 | 0.2074 | 0.2074 |
| corresp. to montelukast acid | 0.1 | 0.1 | 0.2 | 0.2 | 0.2 |

| **Excipients** | | | | | |
|---|---|---|---|---|---|
| Propylene glycol | 5 | 15 | 30 | 40 | 50 |
| Sodium saccharin | 1 | 1 | 1 | 1 | 1 |
| Sodium benzoate | 0.2 | 0.1 | 0.1 | 0.1 | 0.1 |
| Buffer solution (pH 9.0) | q.s.1 ml | q.s.1 ml | q.s.1 ml | q.s.1 ml | q.s.1 ml |
| **Precipitation (3 months)** | Yes | Yes | Yes | Yes | Yes |

**Table 4**

| **Active substance** | % w/v | | | | |
|---|---|---|---|---|---|
| Montelukast sodium | 0.1037 | 0.1037 | 0.2074 | 0.2074 | 0.2074 |
| corresp. to montelukast acid | 0.1 | 0.1 | 0.2 | 0.2 | 0.2 |

| **Excipients** | | | | | |
|---|---|---|---|---|---|
| Ethanol | 5 | 15 | 15 | - | - |
| Glycerol | - | - | 20 | 20 | 40 |
| Sodium saccharin | 1 | 1 | 1 | 1 | 1 |
| Sodium benzoate | 0.2 | 0.1 | 0.1 | 0.1 | 0.1 |
| Buffer solution (pH 9.0) | q.s.1 ml | q.s.1 ml | q.s.1 ml | q.s.1 ml | q.s.1 ml |
| **Precipitation (3 months)** | No | No | No | Yes | Yes |
| **% sulfoxide (initial)** | 0.2 | 0.2 | 0.1 | - | - |
| **% sulfoxide (3 months)** | 1.7 | 1.4 | 1.3 | - | - |
| **% total impurities (initial)** | 0.5 | 0.4 | 0.5 | - | - |
| **% total impurities (3 months)** | 5.4 | 4.4 | 3.1 | - | - |

All resulting solutions were proved to show formation of a white precipitate or high levels of known and unknown impurities after three months of storage under actual in-use conditions at room temperature of 20 - 25°C, indicating that montelukast sodium decomposes in the selected liquid carriers.

Under the above described circumstances the present inventors conducted an extensive research program with view towards solving the stability problems as regards the uncontrolled appearance of degradation products of montelukast sodium. The present inventors developed oral solutions, which excellent stability behavior.

Namely, it has surprisingly been found that a combination of two alcohols, namely propylene glycol and ethanol, in certain concentration ranges along with pH adjustment in the range 7.5 to 9.0 leads to stable montelukast sodium compositions. Importantly, said compositions may be free of additional stabilizing excipients such as antimicrobial agents and antioxidants which may raise additional safety and toxicity issues.

The oral pharmaceutical solutions, according to the present invention, comprise montelukast sodium as an active ingredient and a liquid carrier comprising 15% to 50% of propylene glycol, 2% to 50% of ethanol and a pharmaceutically acceptable buffer, wherein the pH of the solution is from 7.5 to 9.0.

Preferably, the oral pharmaceutical solutions according to the present invention comprise 15% to 30% of propylene glycol. More preferably, the solutions comprise 15% to 25% of propylene glycol.

Preferably, the oral pharmaceutical solutions according to the present invention comprise 2% to 30% of ethanol. More preferably, the solutions comprise 2% to 20% of ethanol.

The oral pharmaceutical solutions according to the present invention preferably comprise montelukast sodium in an amount that corresponds to from 0.05 g to 0.5 g of montelukast acid per 100ml of solution, more preferably to from 0.1 g to 0.25 g of montelukast acid per 100ml of solution.

The oral pharmaceutical solutions, according to the present invention allow the optimal selection of concentration of ethanol and propylene glycol at the lowest feasible level without compromising stability of the solutions.

Any suitable system which acts as a buffer, in the basic pH range of 7.5 to 9.0 can be employed in the compositions of the present invention. Such buffer systems include phosphate buffers such as sodium or potassium monobasic phosphates or dibasic phosphates as well as other pharmaceutically acceptable buffers. Mixtures of buffering agents or other buffering agents can also be used. Preferably, the buffering agent is a phosphate buffer prepared using monosodium dihydrogen phosphate and its conjugate base, disodium phosphate or sodium hydroxide.

Buffer solutions work effectively if they have sufficient buffering capacity to resist the change in pH expected during the production and the storage period. Buffer solutions that are used in the compositions of the present invention should exhibit at least a minimum buffer capacity, so as to achieve pH values in the range of 7.5 to 9.0.

Alcohols and glycols have long been known to exhibit antimicrobial activity. Ethanol is known for being a bactericidal and fungicidal agent while propylene glycol has been defined as a true preservative with fungistatic and fungicidal effect.

Thus, in a preferred embodiment of the invention the oral pharmaceutical solutions of montelukast sodium may be free of additional ingredients commonly used for the preservation of oral liquid pharmaceutical compositions, such as additional antimicrobial agents, and antioxidants, which may raise additional safety and toxicity issues. Absence of additional antimicrobial agents and antioxidants is important particularly in case of paediatric formulations.

The oral pharmaceutical solutions of montelukast sodium according to the invention may also optionally contain additional ingredients commonly used in the preparation of oral liquid pharmaceutical compositions, such as viscosity adjusting agents, non-sugar based artificial sweetening agents, flavoring agents and the like.

Viscosity adjusting agents are used in proportions sufficient to correct or adjust the physical and rheological properties of said compositions. Suitable viscosity adjusting agents include xanthan gum, acacia, guar gum, locust bean gum, gum tragacanth, starch, carbopols, sodium carboxy methylcellulose, methylcellulose, hydroxypropyl cellulose, polyvinylpyrrolidone, glycerin or mixtures thereof.

The non-sugar based artificial sweetening agents which may be used in the present invention include sucralose, saccharin, saccharin sodium, aspartame and acesulfame potassium.

Appropriate flavoring agents may include any of the many non-toxic natural or artificial flavoring agents known in the art. In particular, the flavoring agents used may include one or more of a variety of natural or artificial fruit flavors, including orange, lemon, lime, blueberry, cherry, apple, berry, pineapple, banana, grape, strawberry, watermelon, and kiwi flavorings, among others. Alternatively, or in addition, the flavoring agents may include one or more of natural or artificial vanilla, chocolate, peanut butter, root beer, pistachio, honey and caramel flavorings, among others.

The compositions of the present invention exhibit excellent stability and extended lifetime. Specifically, they remain physicochemically stable when stored for at least three months at temperature of 40°C and relative humidity of 75 per cent (accelerated stability conditions as defined in International Conference on Harmonisation (ICH) guideline named "Stability Testing of New Drug Substances and Products"), with the main impurity (sulfoxide) being less than 0.5% by weight relative to the initial amount of montelukast sodium and with the total impurities being less than 1.0%.

The oral pharmaceutical solutions of montelukast sodium according to the invention also exhibit excellent stability and extended in-use shelf life when supplied in multidose containers. Specifically, they remain physicochemically stable and no precipitate is formed when stored at room temperature of 20°C - 25°C and the containers are uncovered at least once a day for at least three months, with the main impurity (sulfoxide) being less than 0.5% by weight relative to the initial amount of montelukast sodium and with the total impurities being less than 1.0%.

The compositions of the present invention may be prepared following processes well known in the art.

One such process is the following:
1. The buffer solution is prepared and adjusted to the desired pH value.
2. An amount of the buffer solution corresponding to approximately 30% of the final batch volume is added to a mixing vessel (main mixing vessel).
3. Ethanol is transferred in an auxiliary vessel and montelukast sodium is added under continuous stirring until it is completely dissolved.
4. Propylene glycol is added to the auxiliary vessel under continuous stirring.
5. The content of the auxiliary vessel is then transferred to the main mixing vessel, under continuous stirring.
6. If needed, the pH of the batch solution is adjusted to the desired pH value.
7. The volume of the solution is adjusted to the desired batch volume by adding buffer solution (preparation of bulk solution prior to filling).

### EXAMPLES

The following examples show the influence of the proposed co-solvents system on the stability of montelukast sodium. Solutions were prepared by a process where the buffer solution is initially prepared and adjusted to pH 8.0 - 8.5. Then, an amount of the buffer solution corresponding to approximately 30% of the final batch volume is added to a mixing vessel. Ethanol (if present) is transferred to an auxiliary vessel and montelukast sodium is added under continuous stirring until it is completely dissolved. Propylene glycol (if present) is added to the auxiliary vessel under continuous stirring. In case ethanol is not used, montelukast sodium is dissolved in propylene glycol in the auxiliary vessel. The content of the auxiliary vessel is transferred to the main mixing vessel, under continuous stirring. If needed, the pH of the solution is adjusted to 8.0 - 8.5 and buffer solution is added in order to bring the volume to the desired value.

The bulk solution is then filled into glass bottles under nitrogen atmosphere and sealed tightly with a screw cap.

Solutions were subjected to both normal accelerated stability studies and to studies simulating actual in-use conditions, as described in the following examples.

### EXAMPLE 1

The following compositions were studied in relation to their stability at storage conditions of temperature (40°C) and relative humidity (75 per cent) for three months. The quantitative determination of impurities in the prepared compositions, before and after the storage period, was carried out by HPLC.

**Table 5: Compositions with increasing concentration of ethanol**

| | | **Composition** | | | | |
|---|---|---|---|---|---|---|
| | | **A** | **B** | **C** | **D** | **E** |
| **Ingredients** | | **%w/v** | | | | |
| **Active substance** | | | | | | |
| Montelukast sodium | | 0.1037 | 0.2074 | 0.2074 | 0.2074 | 0.2074 |
| corresp. to montelukast acid | | 0.1 | 0.2 | 0.2 | 0.2 | 0.2 |

| **Excipients** | | | | | | |
|---|---|---|---|---|---|---|
| Propylene glycol | | 20 | 20 | 20 | 20 | 20 |
| Ethanol | | - | 2 | 10 | 30 | 50 |
| Buffer solution (pH 8.0) | | q.s. 1 ml | q.s. 1 ml | q.s. 1 ml | q.s. 1 ml | q.s. 1 ml |
| pH | | 8.0 | 8.0 | 8.0 | 8.0 | 8.0 |
| **Sulfoxide %** | **(initial)** | 0.14 | 0.14 | 0.12 | 0.09 | 0.11 |
| | **(3 months)** | 1.8 | 0.44 | 0.31 | 0.33 | 0.40 |
| **Total impurities %** | **(initial)** | 0.46 | 0.48 | 0.39 | 0.41 | 0.31 |
| | **(3 months)** | 5.6 | 0.91 | 0.71 | 0.79 | 0.78 |

**Table 6: Compositions with increasing concentration of propylene glycol**

| | | **Composition** | | | | | |
|---|---|---|---|---|---|---|---|
| | | **F** | **G** | **H** | **I** | **J** | **K** |
| **Ingredients** | | %w/v | | | | | |
| **Active substance** | | | | | | | |
| Montelukast sodium | | 0.1037 | 0.1037 | 0.2074 | 0.2074 | 0.2074 | 0.2074 |
| corresp. to montelukast acid | | 0.1 | 0.1 | 0.2 | 0.2 | 0.2 | 0.2 |

| **Excipients** | | | | | | | |
|---|---|---|---|---|---|---|---|
| Propylene glycol | | - | 5 | 10 | 15 | 30 | 50 |
| Ethanol | | 10 | 10 | 10 | 10 | 10 | 10 |
| Buffer solution (pH 8.0) | | q.s. 1ml | q.s. 1ml | q.s. 1ml | q.s. 1ml | q.s. 1ml | q.s. 1ml |
| pH | | 8.0 | 8.0 | 8.0 | 8.1 | 8.0 | 8.0 |
| **Sulfoxide %** | **(initial)** | 0.12 | 0.10 | 0.09 | 0.11 | 0.12 | 0.09 |
| | **(3 months)** | 2.1 | 1.7 | 0.56 | 0.35 | 0.41 | 0.42 |
| **Total impurities %** | **(initial)** | 0.45 | 0.43 | 0.44 | 0.41 | 0.44 | 0.48 |
| | **(3 months)** | 6.9 | 5.1 | 1.6 | 0.83 | 0.88 | 0.91 |

It was found that the presence of ethanol contributed significantly to the stability of the composition.

Furthermore it was revealed that the gradual increase of the concentration of propylene glycol over 15% significantly contributes to the stability of the composition.

### EXAMPLE 2

The compositions of example 1 were also studied in relation to their in-use stability behavior at storage conditions of 20 - 25°C for three months. The quantitative determination of impurities in the prepared compositions, before and after the storage period, was carried out by HPLC. The possible presence of white precipitation was evidenced by visual inspection over the whole period of three months.

The three-month test simulated the use of the product in practice since the bottles were uncovered once a day and a certain quantity of solution was removed each time. The quantity of removed solution has been chosen so that enough solution for performing all analytical tests at initial and final time points is left in the bottles.

**Table 7: Compositions with increasing concentration of ethanol - in use stability**

| | | **Composition** | | | | |
|---|---|---|---|---|---|---|
| | | **A** | **B** | **C** | **D** | **E** |
| **Ingredients** | | %w/v | | | | |
| **Active substance** | | | | | | |
| Montelukast sodium | | 0.1037 | 0.2074 | 0.2074 | 0.2074 | 0.2074 |
| corresp. to montelukast acid | | 0.1 | 0.2 | 0.2 | 0.2 | 0.2 |

| **Excipients** | | | | | | |
|---|---|---|---|---|---|---|
| Propylene glycol | | 20 | 20 | 20 | 20 | 20 |
| Ethanol | | - | 2 | 10 | 30 | 50 |
| Buffer solution (pH 8.0) | | q.s. 1 ml | q.s. 1 ml | q.s. 1 ml | q.s. 1 ml | q.s. 1 ml |
| pH | | 7.9 | 8.0 | 8.0 | 8.0 | 8.0 |
| **Precipitation** | **(3 months)** | Yes | No | No | No | No |
| **Sulfoxide %** | **(initial)** | - | 0.14 | 0.12 | 0.09 | 0.11 |
| | **(3 months)** | - | 0.46 | 0.41 | 0.45 | 0.48 |
| **Total impurities %** | **(initial)** | - | 0.48 | 0.39 | 0.41 | 0.43 |
| | **(3 months)** | - | 0.97 | 0.82 | 0.92 | 0.86 |

**Table 8: Compositions with increasing concentration of propylene glycol - in use stability**

| | | **Composition** | | | | | |
|---|---|---|---|---|---|---|---|
| | | **F** | **G** | **H** | **I** | **J** | **K** |
| **Ingredients** | | %w/v | | | | | |
| **Active substance** | | | | | | | |
| Montelukast sodium | | 0.1037 | 0.1037 | 0.2074 | 0.2074 | 0.2074 | 0.2074 |
| corresp. to montelukast acid | | 0.1 | 0.1 | 0.2 | 0.2 | 0.2 | 0.2 |

| **Excipients** | | | | | | | |
|---|---|---|---|---|---|---|---|
| Propylene glycol | | - | 5 | 10 | 15 | 30 | 50 |
| Ethanol | | 10 | 10 | 10 | 10 | 10 | 10 |
| Buffer solution (pH 8.0) | | q.s. 1ml | q.s. 1ml | q.s. 1ml | q.s. 1ml | q.s. 1ml | q.s. 1ml |
| pH | | 8.0 | 8.0 | 8.0 | 8.1 | 8.0 | 8.0 |
| **Precipitation** | **(3 months)** | Yes | Yes | Yes | No | No | No |
| **Sulfoxide** % | **(initial)** | - | - | - | 0.11 | 0.12 | 0.13 |
| | **(3 months)** | - | - | - | 0.45 | 0.42 | 0.46 |
| **Total impurities %** | **(initial)** | - | - | - | 0.41 | 0.44 | 0.46 |
| | **(3 months)** | - | - | - | 0.81 | 0.79 | 0.89 |

It was found that the absence of ethanol leads to the formation of white precipitate after three months of in use stability.

Furthermore, it was revealed that concentrations of propylene glycol lower than 15% also lead to the formation of white precipitate.

Although the nature of the precipitation is difficult to be determined, it is easily observed visually without using any microscope.

### EXAMPLE 3

Tables 9 & 10 show preferred compositions of oral solutions, according to the present invention. For minimizing any possible toxicity concerns or patient safety issues, lowest possible concentrations of the selected solvents are utilized.

Typical accelerated (temperature 40°C and relative humidity 75 per cent) and in use stability studies (storage conditions of 20 - 25°C) were applied to both compositions for a period of three months.

**Table 9: Preferred compositions according to the invention**

| | **Composition I** | **Composition II** |
|---|---|---|
| **Ingredients** | % | |
| **Active substance** | | |
| Montelukast sodium | 0.1037 | 0.4148 |
| corresp. to montelukast acid | 0.1 | 0.4 |

| **Excipients** | | |
|---|---|---|
| Propylene glycol | 15 | 15 |
| Ethanol | 2 | 2 |
| Phosphate buffer solution (pH 8.0) | q.s. 1 ml | q.s. 1 ml |
| pH | 8.5 | 8.0 |
| **Sulfoxide % (3 months - accelerated)** | 0.41 | 0.46 |
| **Total impurities % (3 months - accelerated)** | 0.89 | 0.93 |

**Table 10: Preferred compositions according to the invention - in use stability**

| | **Composition I** | **Composition III** |
|---|---|---|
| **Ingredients** | % | |
| **Active substance** | | |
| Montelukast sodium | 0.1037 | 0.4148 |
| corresp. to montelukast acid | 0.1 | 0.4 |

| **Excipients** | | |
|---|---|---|
| Propylene glycol | 15 | 15 |
| Ethanol | 2 | 2 |
| Phosphate buffer solution (pH 8.0) | q.s. 1 ml | q.s. 1 ml |
| pH | 9.0 | 8.0 |
| **Precipitation (3 months - room conditions)** | No | No |
| **Sulfoxide % (3 months - room conditions)** | 0.43 | 0.40 |
| **Total impurities % (3 months - room conditions)** | 0.82 | 0.89 |

## Claims

1. An oral pharmaceutical solution comprising
montelukast sodium,
15 to 50% propylene glycol,
2% to 50% ethanol,
and a pharmaceutically acceptable buffer, wherein the pH of the solution is from 7.5 to 9.0.

2. An oral pharmaceutical solution according to claim 1, comprising 15 to 30% propylene glycol.

3. An oral pharmaceutical solution according to claim 2, comprising 15 to 25% propylene glycol.

4. An oral pharmaceutical solution according to any one of claims 1 to 3, comprising 2% to 30% ethanol.

5. An oral pharmaceutical solution according to any one of claim 4, comprising 2% to 20% ethanol.

6. An oral pharmaceutical solution according to any one of claims 1 to 5, comprising montelukast sodium corresponding to from 0.05% to 0.5% of montelukast acid.

7. An oral pharmaceutical solution according to claim 6, comprising montelukast sodium corresponding to from 0.1% to 0.25% of montelukast acid.

8. An oral pharmaceutical solution according to any one of the preceding claims, wherein the buffer is a phosphate buffer.

9. An oral pharmaceutical solution according to claim 8, wherein the phosphate buffer is a sodium or potassium monobasic or dibasic phosphate buffer.

10. An oral pharmaceutical solution according to claim 1, comprising
montelukast sodium corresponding to 0.1% of montelukast acid,
15% propylene glycol,
2% ethanol,
and a pharmaceutically acceptable buffer, wherein the pH of the solution is 9.0.

11. An oral pharmaceutical solution according to claim 1, comprising
montelukast sodium corresponding to 0.4% of montelukast acid,
15% propylene glycol,
2% ethanol,
and a pharmaceutically acceptable buffer, wherein the pH of the solution is 8.0.

12. An oral pharmaceutical solution according to any one of the preceding claims, which is free of additional antimicrobial agents and/or antioxidants.
